# EUROPEAN PATENT APPLICATION

(11) **EP 1 462 058 A1**
(43) Date of publication of application: **29.09.2004**
(21) Application number: 04002225.3
(22) Date of filing: 02.02.2004
(51) Int. Cl.: A61B 10/00, A61B 5/053

(54) **Female physical condition management apparatus**

(30) Priority: 26.03.2003 JP 2003085031
(71) Applicant: TANITA CORPORATION, Tokyo (JP)
(72) Inventor: Takehara, Tomoko, Itabashi-Ku Tokyo (JP)
(74) Representative: Grünecker, Kinkeldey, Stockmair & Schwanhäusser Anwaltssozietät

(57) **Abstract**

There is provided a female physical condition management apparatus which facilitates a user planning activities, comprising physical condition data acquiring means, appearance time estimation means, estimation accuracy determination means, and display means, wherein the physical condition data acquiring means acquires data about physical conditions of a female which appear in a monthly cycle, the appearance time estimation means estimates the oncoming times of appearance of the physical conditions appearing in a monthly cycle of the female based on the acquired data about the physical conditions, the estimation accuracy determination means determines the accuracy of the estimations made by the appearance period estimation means, and the display means displays data about the estimated times of appearance and data about the determined estimation accuracy. Thus, the female physical condition management apparatus allows the female user to know how reliable the estimation is and facilitates the user planning activities in the future.

## Description

### BACKGROUND OF THE INVENTION

### (i) Field of the Invention

The present invention relates to a female physical condition management apparatus which acquires data about physical conditions of a female which appear in a monthly cycle and estimates the oncoming times of appearance of the physical conditions appearing in a monthly cycle of the female based on the acquired data about the physical conditions.

### (ii) Description of the Related Art

It is beneficial for a female to know in advance the oncoming times of appearance of a physical condition appearing in a monthly cycle of the female such as the start date of menstruation, an ovulation date, a menstrual period, a preovulatory and postovulatory period or a PMS (Premenstrual Syndrome) period, in an attempt to plan activities in the future. For example, since a female generally becomes free of edema, feels that her body is light and is mentally stable during a period from the end of menstruation to around an ovulation date, the female plans active activities such as exercise, diet and traveling, while since the female is liable to have edema on her body, become easily tired and become mentally unstable during a period from the end of ovulation to the end of menstruation, the female refrains from unreasonable exercise, diet or traveling and plans to take a rest instead. Thus, if the female can know the oncoming times of appearance of the physical conditions in advance, she can plan appropriate activities based on the times of appearance.

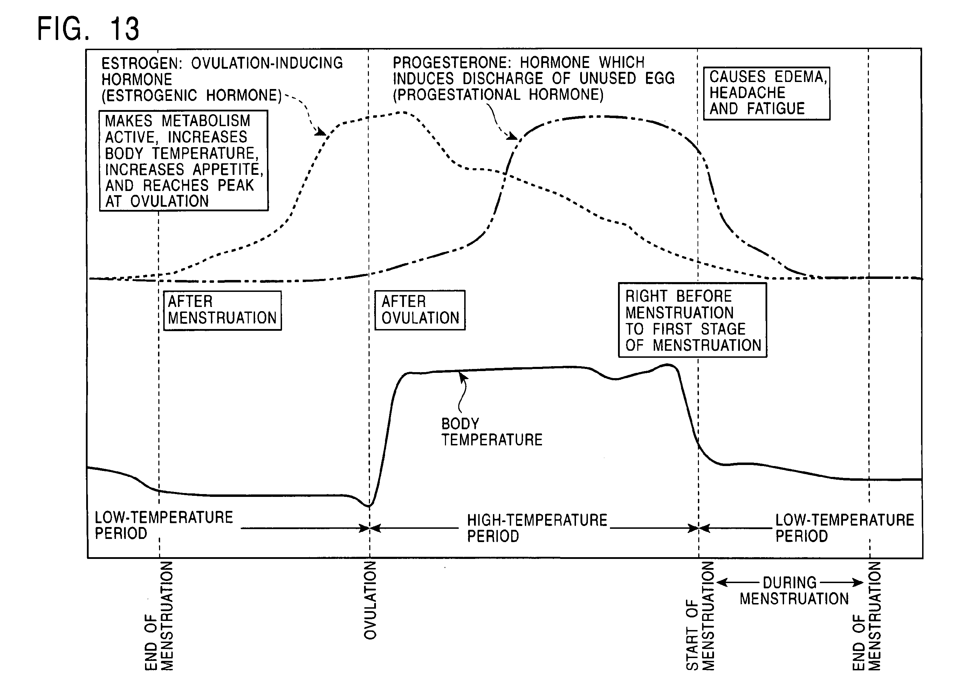

Meanwhile, between physical conditions appearing in a monthly cycle of a female and the basal body temperature of the female, there is a close relationship that the basal body temperature undergoes a transition between a high-temperature period and a low-temperature period on the start date of menstruation and an ovulation date as shown in Fig. 13. Based on the relationship, the current physical condition has been determined and the oncoming times of appearance of the physical conditions have been estimated by measuring and recording the basal body temperature continuously by use of a female basal body thermometer. Further, in recent years, a female basal body thermometer is known which is capable of not only measuring a basal body temperature but also estimating the start date of the next menstruation and the next ovulation date based on transition of the measured basal body temperatures and data about the start date of menstruation input by a user and displaying the estimated data (for example, refer to non-patent publication 1).

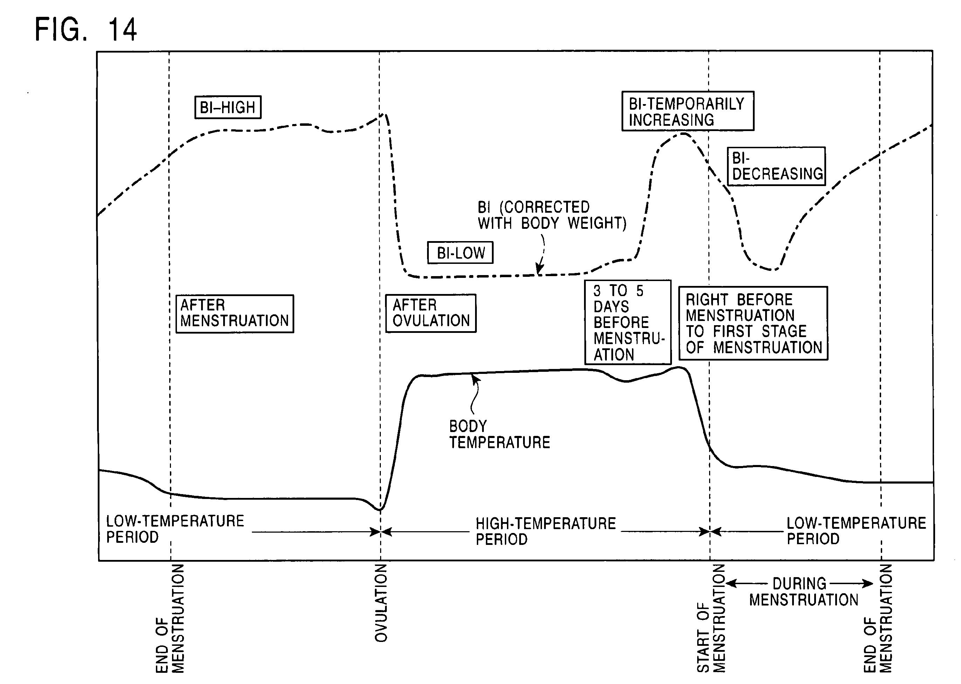

Further, the present applicant has focused attention on a fact that between the bioelectric impedance (hereinafter may be abbreviated as "BI" in the present specification) of a female and her basal body temperature, there is a relationship that the BI value is high during the low temperature period of the basal body temperature and the BI value is low during the high temperature period of the basal body temperature as shown in Fig. 14 and has already proposed a female physical condition management apparatus which measures the BI value of a female and determines which of physical conditions appearing in a monthly cycle such as a menstrual period, a preovulatory and a PMS period the current physical condition of the female corresponds to based on transition of the measured BI values and a female physical condition management apparatus which estimates and displays the times of appearance of specific physical conditions such as the start date of the next menstruation, ovulation date, fertile period, and period suitable for diet (for example, refer to patent publications 1 and 2).

### Non-Patent Publication 1

Terumo Corporation, "LUNACHECK II Terumo electronic thermometer C595 instruction manual", first printing in September 2000, pp. 15 to 17, [online], homepage of Terumo Corporation Healthcare Information Office LUNACHECK instruction manual "LUNACHECK II Terumo electronic thermometer C595", "Searched on August 9, 2002", Internet <URL:http://www.terumo.co.jp/healthcare/241/ETC595.PDF>

Attention: the above URL has already been changed to "URL:http://www.terumo.co.jp/healthcare/qa/pdf/ET_C595.pdf " as of now, November 6, 2003.

### <Patent Publication 1>

Japanese Patent Application Laid-Open No. 78977/2001

### <Patent Publication 2>

Japanese Patent Application Laid-Open No. 102192/2002

In the above conventional female basal body thermometer and female physical condition management apparatuses, the start date of the next menstruation, the next ovulation date or the times of appearance of physical conditions appearing in a monthly cycle such as a menstrual period, a preovulatory and postovulatory period, a PMS period, a fertile period and a period suitable for diet are estimated and displayed on a display screen. However, despite the fact that the displayed times of appearance of the physical conditions are mere estimations, these conventional female basal body thermometer and female physical condition management apparatuses do not display any data about the accuracy of the estimations. Thus, users do not know how reliable the displayed times of appearance are or how much they can rely on the displayed data, so that it is still difficult for the users to plan activities in the future.

Further, in the above conventional female basal body thermometer and female physical condition management apparatuses, the above estimations of the times of appearance are made based on the start dates of menstruation entered in these apparatuses or transitions of data such as a basal body temperature or bioelectric impedance measured by these apparatuses. Hence, the accuracy of the above estimations improves as the above data is acquired continuously over a long period of time. In other words, while the number of times the above data has been acquired is still small due to the reason that these apparatuses have just recently started to be used, the above estimation accuracy is low. However, users are apt to thoroughly believe the estimated times of appearance especially during an early stage of use of these apparatuses. Thus, if the estimations fail during such an early stage, the users may stop using these apparatuses continuously due to disappointment, so that the number of times the above data has been acquired hardly increases and the above estimation accuracy does not improve.

Therefore, an object of the present invention is to provide a female physical condition management apparatus which estimates the times of appearance of physical conditions of a female which appear in a monthly cycle, whereby the above problems of the prior art are solved and a user can know the accuracy of the estimations so as to facilitate planning activities in the future.

Further, another object of the present invention is to provide a female physical condition management apparatus which estimates the times of appearance of physical conditions of a female which appear in a monthly cycle, whereby the above problems of the prior art are solved and a user is induced to continue to use the apparatus until the accuracy of the estimations improves.

### SUMMARY OF THE INVENTION

A female physical condition management apparatus of the present invention comprises:
physical condition data acquiring means,
appearance time estimation means,
estimation accuracy determination means, and
display means,
wherein
the physical condition data acquiring means acquires data about physical conditions of a female which appear in a monthly cycle, the appearance time estimation means estimates the oncoming times of appearance of the physical conditions appearing in a monthly cycle of the female based on the acquired data about the physical conditions,
the estimation accuracy determination means determines the accuracy of the estimations made by the appearance period estimation means, and
the display means displays data about the estimated times of appearance and data about the determined estimation accuracy.

Further, in the female physical condition management apparatus of the present invention, the estimation accuracy determination means determines the accuracy of the estimations made by the appearance time estimation means based on the number of times the data about the physical conditions appearing in a monthly cycle of the female has been acquired by the physical condition data acquiring means in the past.

Further, in the female physical condition management apparatus of the present invention, the data about the estimation accuracy which is displayed by the display means is a probability that the physical conditions appearing in a monthly cycle of the female actually appear at the estimated times of appearance.

Further, in the female physical condition management apparatus of the present invention, the physical condition data acquiring means is used by the female to input the data about the physical conditions appearing in a monthly cycle.

Further, in the female physical condition management apparatus of the present invention, the physical condition data acquiring means measures physical data of the female and acquires the data about the physical conditions appearing in a monthly cycle of the female based on the measured physical data.

Further, in the female physical condition management apparatus of the present invention, the physical data of the female is the basal body temperature of the female.

Further, in the female physical condition management apparatus of the present invention, the physical data of the female is the bioelectric impedance of the female.

Further, in the female physical condition management apparatus of the present invention, the data about the physical conditions appearing in a monthly cycle of the female which is acquired by the physical condition data acquiring means is the start date of menstruation of the female.

Further, in the female physical condition management apparatus of the present invention, the data about the physical conditions appearing in a monthly cycle of the female which is acquired by the physical condition data acquiring means is the ovulation date of the female.

Further, in the female physical condition management apparatus of the present invention, the data about the physical conditions appearing in a monthly cycle of the female which is acquired by the physical condition data acquiring means is the number of days in the monthly cycle of the female.

### BRIEF DESCRIPTION OF THE DRAWINGS

Fig. 1 is a diagram showing the external appearance of a female physical condition management apparatus according to the present invention.
Fig. 2 is a block diagram showing the constitution of functions of the female physical condition management apparatus according to the present invention.
Fig. 3 is a flowchart showing the main processing of the female physical condition management apparatus according to the present invention.
Fig. 4 is a flowchart showing the input process of the female physical condition management apparatus according to the present invention.
Fig. 5 is a flowchart showing the measuring process of the female physical condition management apparatus according to the present invention.
Fig. 6 is a flowchart showing the estimation process of the female physical condition management apparatus according to the present invention.
Fig. 7 is a diagram showing probability data stored in the female physical condition management apparatus according to the present invention.
Fig. 8 is a diagram showing an initial screen display of the female physical condition management apparatus according to the present invention.
Figs. 9A and 9B are diagrams showing screen displays of the female physical condition management apparatus according to the present invention.
Figs. 10A and 10B are diagrams showing other screen displays of the female physical condition management apparatus according to the present invention.
Fig. 11 is a screen display transition diagram of the female physical condition management apparatus according to the present invention.
Figs. 12A and 12B are diagrams showing still other screen displays of the female physical condition management apparatus according to the present invention.
Fig. 13 is a diagram showing a relationship among a physical condition of a female which appears in a monthly cycle, a basal body temperature, and hormone secretion.
Fig. 14 is a diagram showing a correlation between bioelectric impedance and a basal body temperature.

### DETAILED DESCRIPTION OF THE PREFERRED EMBODIMENTS

The female physical condition management apparatus of the present invention comprises physical condition data acquiring means, appearance time estimation means, estimation accuracy determination means, and display means, wherein the physical condition data acquiring means acquires data about physical conditions of a female which appear in a monthly cycle, the appearance time estimation means estimates the oncoming times of appearance of the physical conditions appearing in a monthly cycle of the female based on the acquired data about the physical conditions, the estimation accuracy determination means determines the accuracy of the estimations made by the appearance period estimation means, and the display means displays data about the estimated times of appearance and data about the determined estimation accuracy. Thus, the female physical condition management apparatus allows the female user to know how reliable the estimation is and facilitates the user planning activities in the future.

Further, the estimation accuracy determination means desirably determines the accuracy of the estimations made by the appearance time estimation means based on the number of times the data about the physical conditions appearing in a monthly cycle of the female has been acquired by the physical condition data acquiring means in the past. Thereby, the estimation accuracy improves as the number of times the data about the physical conditions has been acquired increases. This can cause the female user to feel that repeated use of the female physical condition management apparatus is advantageous and enjoyable and to have the feeling of using the apparatus continuously.

Further, the data about the estimation accuracy which is displayed by the display means is desirably a probability that the physical conditions appearing in a monthly cycle of the female actually appear at the estimated times of appearance. Thereby, the female user can easily and clearly know how reliable the estimation is and plan activities in the future more easily.

Further, the physical condition data acquiring means may be used by the female to input the data about the physical conditions appearing in a monthly cycle. In this case, the female user uses the data about the physical conditions which has been acquired by use of, for example, a known female basal body thermometer in this female physical condition management apparatus so as to know the oncoming times of appearance of the physical conditions appearing in a monthly cycle and estimation accuracy thereof.

Alternatively, the physical condition data acquiring means may measure physical data of the female and acquire the data about the physical conditions appearing in a monthly cycle of the female based on the measured physical data. In this case, the female user can acquire the data about the physical conditions by the female physical condition management apparatus and know the oncoming times of appearance of the physical conditions appearing in a monthly cycle and estimation accuracy thereof.

Particularly, the physical data of the female may be the basal body temperature or bioelectric impedance of the female. The data about the physical conditions appearing in a monthly cycle of the female can be acquired regardless of whether the physical data is the basal body temperature or the bioelectric impedance. The oncoming times of appearance of the physical conditions appearing in a monthly cycle of the female can be estimated and the accuracy of the estimation can be determined based on the physical condition data.

Further, the data about the physical conditions appearing in a monthly cycle of the female which is acquired by the physical condition data acquiring means may be the start date of menstruation, ovulation date or menstrual cycle of the female. The oncoming times of appearance of the physical conditions appearing in a monthly cycle of the female can be estimated and the accuracy of the estimation can be determined based on the physical condition data, regardless of whether the physical condition data is the start date of menstruation, the ovulation date or the menstrual cycle.

### Examples

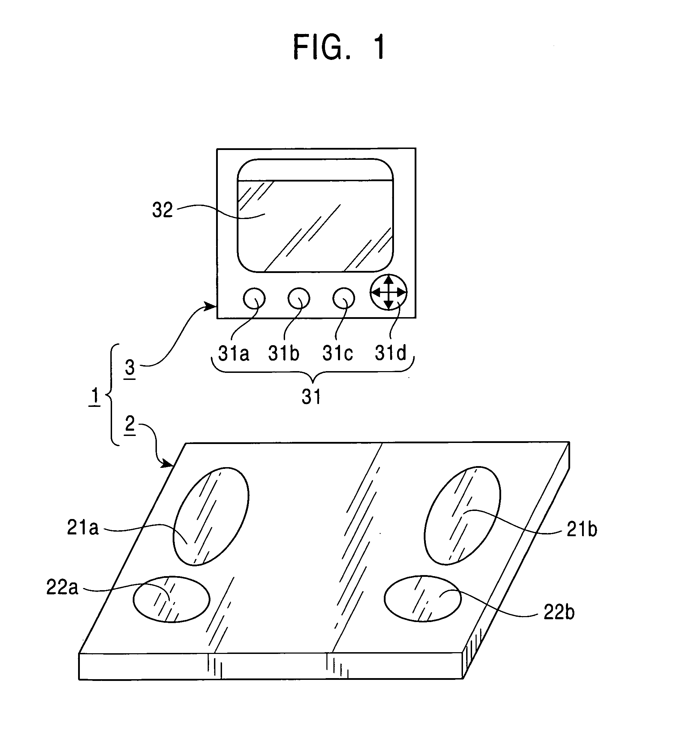

A suitable embodiment of the present invention will be described by use of the drawings hereinafter. Fig. 1 is a diagram showing the external appearance of a female physical condition management apparatus 1 according to the present invention. As shown in Fig. 1, the female physical condition management apparatus 1 comprises a BI measuring device 2 that is equipped with a scale and can measure a body weight and a BI value as physical data of a user and a control box 3 that is connected to the BI measuring device 2 having a scale via a wireless communication by, for example, infrared or radio waves. Although the BI measuring device 2 having a scale and the control box 3 in the present embodiment are connected to each other via wireless communication by, for instance, infrared or radio waves, they may be connected to each other via a commonly used electric cable.

Provided on the top surface of the BI measuring device 2 having a scale are constant current applying electrodes 21a and 21b and voltage measuring electrodes 22a and 22b which make contact with the bottoms of both feet of a user. Further, on the front surface of the control box 3, an operation section 31 and a display screen 32 are provided. The operation section 31 comprises a power button 31a for turning on/off the female physical condition management apparatus 1, an input mode button 31b for selecting a mode (to be described later) of inputting data about physical conditions appearing in a monthly cycle, a measuring mode button 31c for selecting a mode (to be described later) of measuring a body weight and a BI value, and a direction button 31d for carrying out, e.g., switching of screen images (to be described later). The display screen 32 displays data about the times of appearance of the physical conditions appearing in a monthly cycle which are estimated by the female physical condition management apparatus 1 and determined estimation accuracy. The direction button 31d has a structure that an UP direction button (↑) , a DOWN direction button (↓), a LEFT direction button (←) and a RIGHT direction button (→) are integrated.

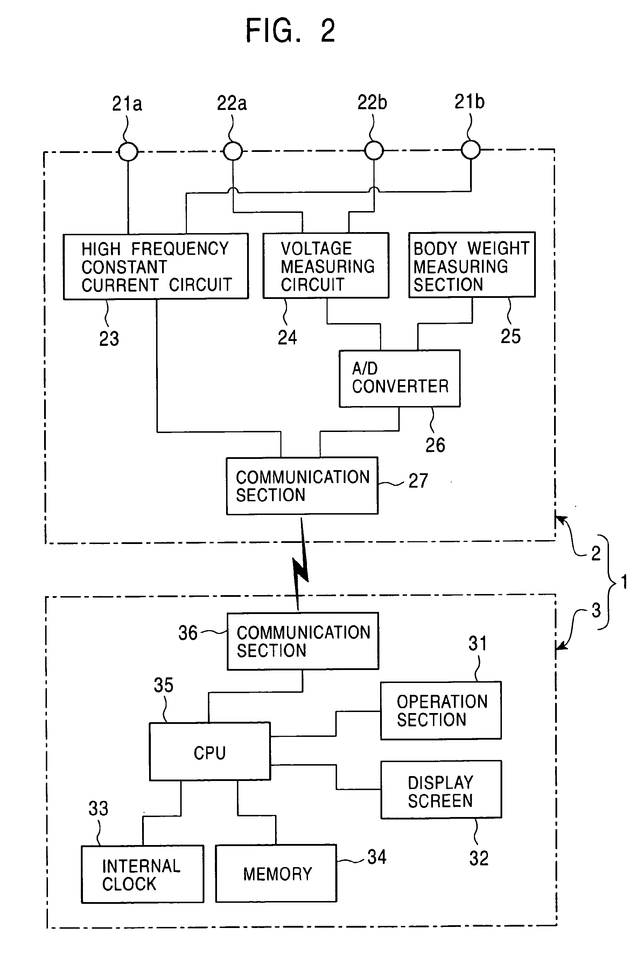

Fig. 2 is a block diagram showing the constitution of functions of the female physical condition management apparatus 1. As shown in Fig. 2, the BI measuring device 2 comprises the constant current applying electrodes 21a and 21b, a high frequency constant current circuit 23 for generating a faint high frequency constant current to be applied to these electrodes 21a and 21b, the voltage measuring electrodes 22a and 22b, a voltage measuring circuit 24 for measuring a voltage between these electrodes 22a and 22b, a body weight measuring section 25 for measuring the body weight of a user, an A/D converter 26 for converting a measured voltage and a measured body weight in analog form to their digital counterparts, and a communication section 27 for wireless communication.

Meanwhile, the control box 3 comprises, in addition to the above operation section 31 and display screen 32, an internal clock 33 for clocking a date and time of use, a memory 34 for storing input data about physical conditions appearing in a monthly cycle, a measured body weight and a measured BI value, the estimated times of appearance of the physical conditions, determined estimation accuracy and other data, a CPU 35 for estimating the times of appearance of the physical conditions appearing in a monthly cycle of the user by processing the input data about the physical conditions appearing in a monthly cycle and data such as the measured body weight and BI value and determining the accuracy of the estimation, and a communication section 36 for wireless communication. In addition to the above estimation of the time of appearance and determination of the accuracy of the estimation, the CPU 35 also displays the results of the estimation and determination on the above display screen 32, stores the data in the above memory 34, and obtains a BI value by processing a voltage value measured in the above voltage measuring circuit 24.

In the present embodiment, the functional elements are classified into either the BI measuring device 2 with a scale or the control box 3 as described above. The present invention is not limited to this particular constitution. For example, the memory 34 and the CPU 35 may be incorporated in the BI measuring device 2 in place of the control box 3. The BI measuring device 2 and the control box 3 may be integrated.

Next, the operation of the female physical condition management apparatus 1 will be described in detail.

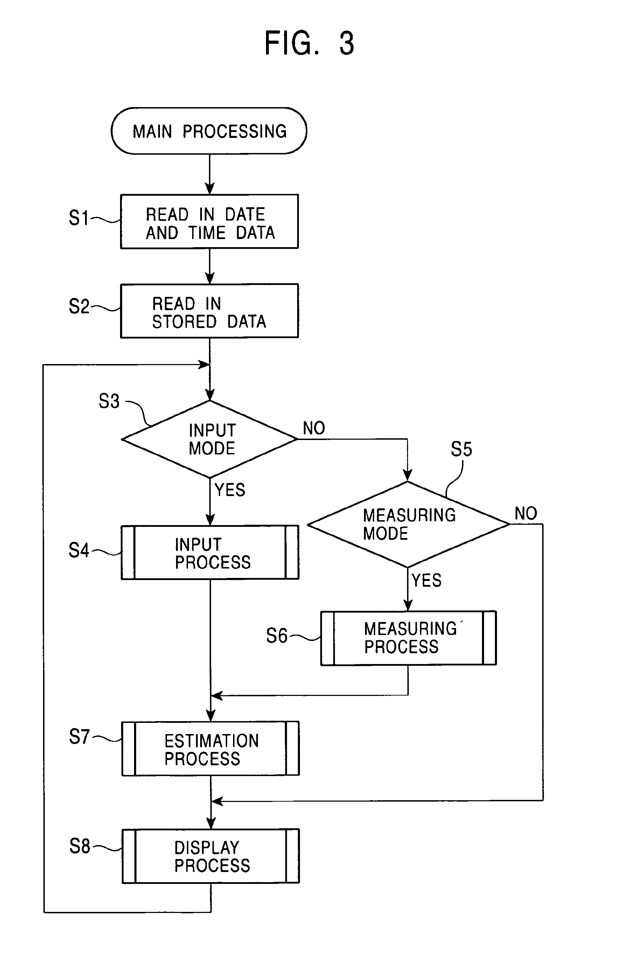

Fig. 3 is a flowchart showing the main processing under the control of the CPU 35. When a user presses down the power button 31a so as to turn on the female physical condition management apparatus 1, the CPU 35 reads in a date and time being clocked by the internal clock 33 so as to gain the current date and time in STEP 1 (hereinafter abbreviated as "S1"). Then, in S2, the CPU 35 reads in data which have been updated through subsequent steps including S3 to be described later and stored in the memory 34 at the last time the user used the female physical condition management apparatus 1, i.e., data about physical conditions appearing in a monthly cycle of the user, data about the estimated times of appearance of the physical conditions, data about the accuracy of the estimation and data such as a measured body weight and a measured BI value (hereinafter in the present embodiment, these data may be collectively referred to as "stored data") and prepares for subsequent processes. Meanwhile, when the user uses the female physical condition management apparatus 1 for the first time, the stored data do not exist, and no data is therefore read in the CPU 35 in S2.

Then, in S3, a determination whether the input mode button 31b has been pressed, i. e. a determination whether an input mode has been selected is made. When the input mode is selected, the CPU 35 proceeds from S3 to S4 where a process (to be described later) of inputting data about the physical conditions appearing in a monthly cycle is executed. Meanwhile, when the input mode is not selected in S3, the CPU 35 proceeds to S5. In S5, a determination whether the measuring mode button 31c has been pressed, i.e. a determination whether a measuring mode has been selected is made. When the measuring mode is selected, the CPU 35 proceeds from S5 to S6 where a process (to be described later) of measuring a body weight and a BI value is executed.

After the input mode is selected in S3 or the measuring mode is selected in S5 and the input process in S4 or the measuring process in S6 is executed, the CPU 35 proceeds to S7. In S7, the times of appearance of the physical conditions appearing in a monthly cycle are estimated, and the accuracy of the estimation is determined as will be described later (estimation process). Meanwhile, when the input mode is not selected in S3 and the measuring mode is not selected in S5, the CPU 35 proceeds from S5 to S8. The CPU 35 also proceeds to S8 after the input mode or the measuring mode is selected and the estimation process in S7 is completed. In S8, the data read from the memory 34 in S2 as stored data or data about the time of appearance and estimation accuracy obtained by the estimation process in S7 are displayed on the display screen 32 as will be described later (displaying process). In the main processing, the processes of S3 to S8 are repeated until the power button 31a is pressed down again so as to turn off the female physical condition management apparatus 1. To turn off the female physical condition management apparatus 1, a so-called "auto power off" function which automatically turns off the female physical condition management apparatus 1 if the operation section 31 is left unoperated at least for a given amount of time may be imparted to the female physical condition management apparatus 1.

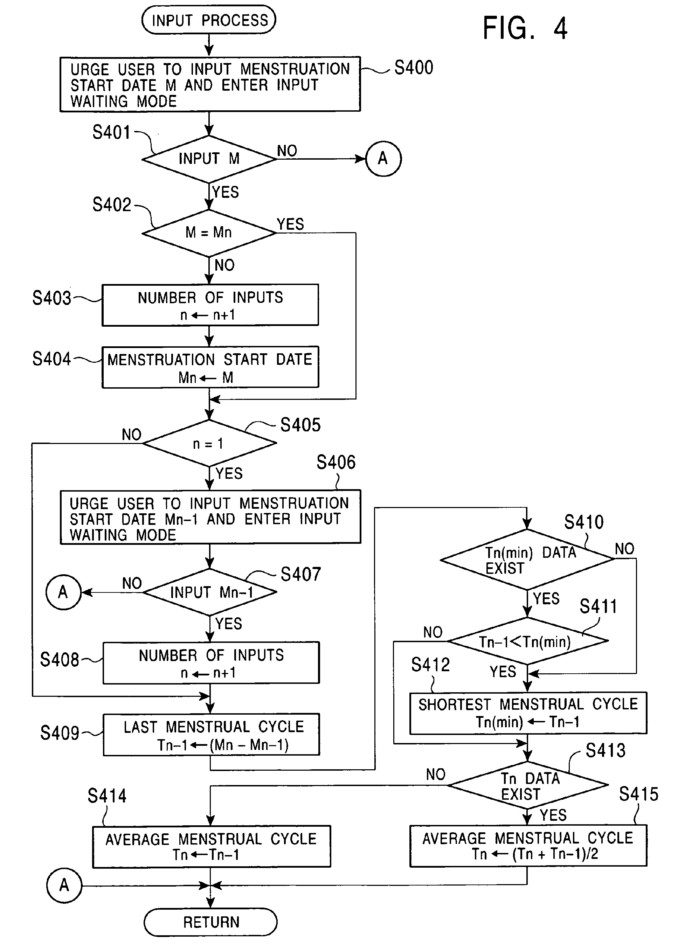

Next, the input process in S4 will be described by use of the flowchart of Fig. 4. In this input process, the user is urged to input the start date of menstruation as data about the physical conditions appearing in a monthly cycle, and the menstrual cycle (number of days from the start date of menstruation to the start date of the next menstruation) of the user is determined from the input start date. Firstly, when the input mode is selected in the main processing, the CPU 35 enters an input waiting mode that lasts for a given time (for example, about 1 minute) while displaying a message such as "Input the start date of the last menstruation." on the display screen 32 so as to urge the user to input the start date M of the most recent menstruation in S400. During the given time, the CPU 35 determines in S401 whether the menstruation start date M has been input. When the menstruation start date M has been input within the given time, the CPU 35 proceeds to S402, while when the menstruation start date M has not been input, the CPU 35 cancels all subsequent steps including S402 and returns to the main processing.

In S402, the CPU 35 compares the input menstruation start date M with a menstruation start date Mₙ read from the memory 34 in S2 of the main processing as stored data and proceeds to S403 if they are different dates. Then, after increasing the number of times n the start date of menstruation has been input (hereinafter referred to as "menstruation input number n" in the present embodiment) by 1 in S403, the CPU 35 updates the menstruation start date Mₙ with the input menstruation start date M in S404 and proceeds to S405. Meanwhile, when the input menstruation start date M and the menstruation start date Mₙ read in S2 are determined to be the same date, the CPU 35 skips S403 and S404 and proceeds to S405. That is, when the dates are found to be different in S402, it implies that the menstrual cycle has entered a new cycle. Thus, the menstruation input number n is increased by 1, and the menstruation start date Mₙ is updated with the input menstruation start date M. However, when the two dates are the same, it implies that the user is still in the same menstrual cycle, so that neither an increase of the menstruation input number n nor an update on the menstruation start date Mₙ is performed. When this input process is executed for the first time, e.g., when the user uses the female physical condition management apparatus 1 for the first time, the menstruation start date Mₙ does not exist, so that the two dates are determined to be different in S402. Hence, the CPU 35 proceeds to S403 where the menstruation input number n is set as 1 and to S404.

Then, in S405, it is determined whether the menstruation input number n is 1. When the menstruation input number n is 1, the menstrual cycle cannot be determined. Thus, the CPU 35 proceeds to S406 and enters an input waiting mode that lasts for a given time (for example, about 1 minute) while displaying a message such as "Input the start date of the last menstruation." on the display screen so as to urge the user to input the menstruation start date Mₙ₋₁ of the last cycle. During the given time, the CPU 35 determines in S407 whether the start date Mₙ₋₁ of the last menstruation has been input. When the start date Mₙ₋₁ of the last menstruation has been input within the given time, the CPU 35 proceeds to S408 so as to increase the menstruation input number n by 1 (resulting in n = 2) and then proceeds to S409, when the menstruation start date Mₙ₋₁ has not been input, the CPU 35 cancels all subsequent steps including S408 and returns to the main processing. Meanwhile, when the menstruation input number n is determined to be 2 or larger in S405, the menstrual cycle can be determined. Thus, the CPU 35 skips S406 to S408 and proceeds to S409.

Then, in S409, a difference in the number of days between the menstruation start date Mₙ of the present cycle and the menstruation start date Mₙ₋₁ of the last cycle is determined, and the last menstrual cycle Tₙ₋₁ is updated with the difference. The menstruation start date Mₙ₋₁ of the last cycle is read from the memory 34 in S2 of the main processing as stored data or input in S407.

Then, in S410 to S412, the shortest menstrual cycle Tₙ ₍ₘᵢₙ₎ is updated. The shortest menstrual cycle Tₙ₍ₘᵢₙ₎ is the shortest menstrual cycle out of menstrual cycles obtained by executing the input process in the past, and it is read from the memory 34 in S2 of the main processing as stored data. Firstly, in S410, it is determined whether data about the shortest menstrual cycle Tₙ₍ₘᵢₙ₎ already exists. At this time, when this input process is executed for the first time, e.g., when the user uses the female physical condition management apparatus 1 for the first time, the data about the shortest menstrual cycle Tₙ₍ₘᵢₙ₎ does not yet exist. In this case, the CPU 35 proceeds from S410 to S412 so as to update the shortest menstrual cycle Tₙ₍ₘᵢₙ₎ with the last menstrual cycle Tₙ₋₁ updated in S409 and then proceeds to S413. Meanwhile, when the data about the shortest menstrual cycle Tₙ₍ₘᵢₙ₎ is determined to already exist in S410, the CPU 35 proceeds to S411. In S411, the last menstrual cycle Tₙ₋₁ updated in S409 is compared with the shortest menstrual cycle Tₙ₍ₘᵢₙ₎. When the last menstrual cycle Tₙ₋₁ is smaller than the shortest menstrual cycle Tₙ₍ₘᵢₙ₎, the CPU 35 proceeds to S412 so as to update the shortest menstrual cycle Tₙ₍ₘᵢₙ₎ with the last menstrual cycle Tₙ₋₁ and then proceeds to S413, while when the last menstrual cycle Tₙ₋₁ is larger than or equal to the shortest menstrual cycle Tₙ₍ₘᵢₙ₎, the CPU 35 proceeds to S413 without going through S412, i.e., without updating the shortest menstrual cycle Tₙ₍ₘᵢₙ₎.

Then, in S413 to S415, an average menstrual cycle Tₙ is updated. The average menstrual cycle Tₙ is an average of menstrual cycle data obtained by executing the input process in the past and is read from the memory 34 in S2 of the main processing as stored data. Firstly, in S413, it is determined whether data about the average menstrual cycle Tₙ exists. At this time, when this input process is executed for the first time, e.g., when the user uses the female physical condition management apparatus 1 for the first time, the data about the average menstrual cycle Tₙ does not yet exist. In this case, the CPU 35 proceeds from S413 to S414 so as to update the average menstrual cycle Tₙ with the last menstrual cycle Tₙ₋₁ updated in S409 and then returns to the main processing. Meanwhile, when the data about the average menstrual cycle Tₙ is determined to already exist in S413, the CPU 35 proceeds to S415 so as to update the average menstrual cycle Tₙ with a value obtained by dividing the sum of the average menstrual cycle Tₙ and the last menstrual cycle Tₙ₋₁ updated in S409 by 2 and then returns to the main processing.

By the above S400 to S415, the menstruation start date and menstrual cycle of the user are obtained as data about the physical conditions appearing in a monthly cycle. Further, the number of times the data about the physical conditions has been obtained is counted. In addition, although in the present embodiment the user inputs the menstruation start date and the menstrual cycle is calculated from the input menstruation start date, she may input an ovulation date or the menstrual cycle itself in place of the menstruation start date.

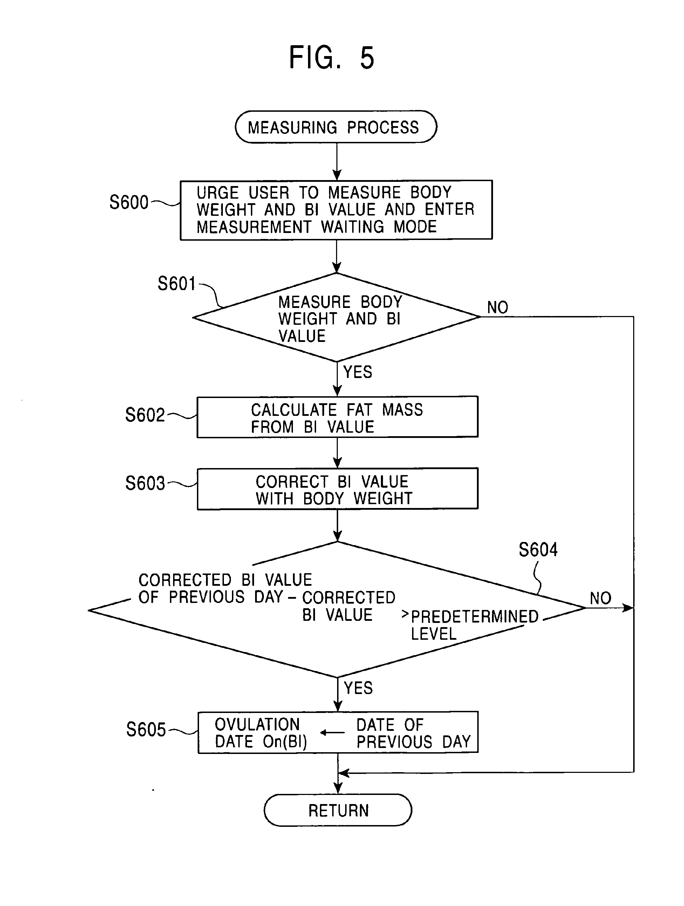

Next, the measuring process in S6 of the main processing will be described by use of the flowchart of Fig. 5. In this measuring process, a body weight and BI value are measured as physical data of the user, and a body fat mass and an ovulation date are determined. Firstly, when the measuring mode is selected in the main processing, the CPU 35 enters a measurement waiting mode that lasts for a given time (for example, about 1 minute) while displaying a message such as "Stand on the measuring platform with the bottoms of your feet placed in the prescribed positions." on the display screen so as to urge the user to measure her body weight and BI value in S600. During the given time, the CPU 35 determines in S601 whether the body weight and the Bi value have been measured. When their measurements have been made within the given time, the CPU 35 proceeds to S602. Meanwhile, when the measurements have not been made, the CPU 35 skips all subsequent steps including S602 and returns to the main processing. To measure the body weight and BI value of the user, the user stands on the top surface of the BI measuring device 2 having a scale with the toe side of the bottom of the left foot in contact with the constant current applying electrode 21a, the heel side of the bottom of the left foot in contact with the voltage measuring electrode 22a, the toe side of the bottom of the right foot in contact with the constant current applying electrode 21b, the heel side of the bottom of the right foot in contact with the voltage measuring electrode 22b.

Then, in S602, the body fat mass of the user is calculated based on the measured BI value. To calculate the body fat mass based on the BI value, the CPU 35 executes given regression calculation program software. A description of the software is omitted in the present specification, since such an apparatus and a method used for the calculation of the body fat mass are publicly well known.

Then, in S603, the measured BI value is corrected by, for example, the following correction formula 1 or correction formula 2 so as to obtain a corrected BI value. A and B in the correction formulae are correction coefficients. The correction of the BI value is made in order to eliminate the influence of a change in the body weight on a change in the BI value when the physical conditions appearing in a monthly cycle of the female are determined based on the BI value and is already known in the above Japanese Patent Application Laid-Open No. 78977/2001 and other publications. Therefore, a detailed description thereof is omitted in the present specification. Correction Formula 1: Body Weight Correction BI = BI + A X Difference from Body Weight Measured for the First Time Correction Formula 2: Body Weight Correction BI = BI + B X Difference from Body Weight Measured the Last Time

Then, in S604, the corrected BI value obtained in S603 is compared with a corrected BI value obtained when the user has executed the measuring mode by use of the female physical condition management apparatus 1 on the previous day and read from the memory 34 in S2 of the main processing as stored data, and it is determined whether the corrected BI value is lower than the corrected BI value obtained on the previous day by at least a given level (for example, at least 4%). As shown in Fig. 14, when the corrected BI value drops sharply by at least the given level, it is assumed that the user is undergoing a change in physical condition due to ovulation. Thus, the CPU 35 proceeds to S605 so as to update the date of an ovulation determination date O_{n(BI)} based on the BI value with the date of the previous day, and then returns to the main processing. Meanwhile, when the corrected BI value is determined to be not lower than the corrected BI value obtained on the previous day by at least the given level in S604, the CPU 35 skips S605 and returns to the main processing. Further, the CPU 35 also returns to the main processing from S604 when the corrected BI value obtained on the previous day does not yet exist, e.g., when the user uses the female physical condition management apparatus 1 for the first time. Although the corrected BI value is compared with the corrected BI value obtained on the previous day in the present embodiment, it may be compared with an average of corrected BI values in a period when the corrected BI values are high in a menstrual cycle.

By the above S600 to S605, the body weight and BI value of the user are measured, and a body fat mass and an ovulation date are determined. In the present embodiment, the ovulation date is determined from transition of the measured BI values. Alternatively, the end date of menstruation or the onset date or end date of edema or PMS may be determined. Further, in the present embodiment, the body fat mass of the user is calculated from the measured BI value. Alternatively, physical components such as a body water content, a basal metabolic rate, a bone density and the amount of muscle may be determined.

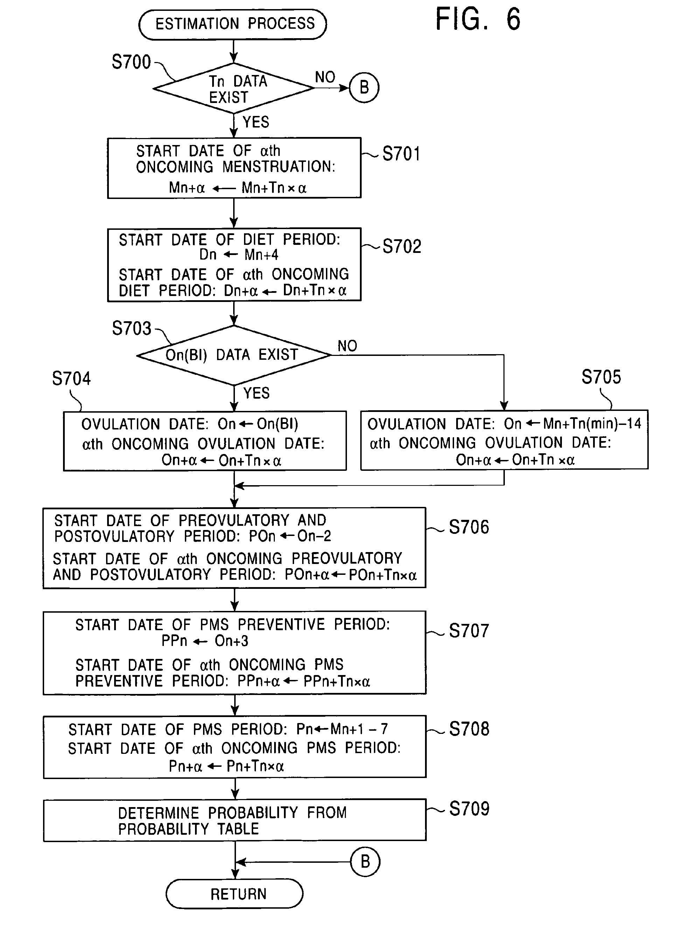

Next, the estimation process in S7 of the main processing will be described by use of the flowchart of Fig. 6. In this estimation process, the times of appearance of physical conditions appearing in a monthly cycle in the present and subsequent cycles are estimated from data about the physical conditions appearing in a monthly cycle, e.g., the menstruation start date Mₙ, average menstrual cycle Tₙ and ovulation date O_{n(BI)} obtained in S4 or S6 of the main processing. In particular, in the present embodiment, as the times of appearance of the physical conditions appearing in a monthly cycle, the start dates of a menstrual period, period suitable for diet (hereinafter abbreviated as "diet period" in the present embodiment), preovulatory and postovulatory period, PMS preventive period and PMS period are estimated. Further, the accuracy of the estimations is also determined.

Firstly, in S700, it is determined whether data about the average menstrual cycle Tₙ exists. When the menstruation input number n counted in the above input process (S403 or S408) of S4 is 2 or larger, it implies that the average menstrual cycle Tₙ exists. Thus, it may be determined in S700 whether the menstruation input number n is 2 or larger instead. When the data about the average menstrual cycle Tₙ does not yet exist, e.g., when the user uses the female physical condition management apparatus 1 for the first time, the CPU 35 skips all subsequent steps and returns to the main processing.

Meanwhile, when the data about the average menstrual cycle Tₙ is determined to exist in S700, the CPU 35 proceeds to S701 so as to estimate the start date M_{n+α} of the α^{th} oncoming menstruation. The start date M_{n+α} of the α^{th} oncoming menstruation is estimated by multiplying the average menstrual cycle Tₙ in days by α and adding the product to the start date Mₙ of menstruation of the present cycle. In this female physical condition management apparatus 1, the value of α is set at 1 to 12. Thus, the female physical condition management apparatus 1 is capable of estimating the start dates of the 1^{st} to 12^{th} oncoming menstruation, i.e., the start dates of menstruation approximately over coming one year. As a matter of course, the maximum value of α is not limited to 12 and may be set at a given value.

Then, in S702, the start date of the diet period Dₙ is estimated. For dieting, a period ranging from the time when basal metabolism starts to increase after the end of a menstrual period to the start of the preovulatory and postovulatory period is suitable. In this female physical condition management apparatus 1, four days including the start date of menstruation Mₙ are set as a menstrual period. Thus, the date following the end of the menstrual period can be estimated as the start date of the diet period Dₙ. That is, in S702, a date obtained by adding four days to the start date Mₙ of menstruation of the present cycle is estimated as the start date Dₙ of the diet period in the present cycle. Then, the start date D_{n+α} of the α^{th} oncoming diet period is estimated by multiplying the average menstrual cycle Tₙ in days by α and adding the product to the start date Dₙ of the diet period of the present cycle. In the present embodiment, although four days including the start date of menstruation Mₙ are set as a menstrual period, up to about 7 days may be set as the menstrual period as appropriate. Further, it is also possible that a period ranging, for example, from the start date of menstruation Mₙ and the time when the BI value becomes high and stable is calculated in each cycle as an actual menstrual period based on transition of the BI value measured in the above measuring process of S6 and an average number of days thereof is taken as the menstrual period.

Then, in S703 to S705, the ovulation date Oₙ is estimated. Firstly, in S703, it is determined whether data about the ovulation determination date O_{n(BI)} based on the BI value updated in the above measuring process (S605) of S6 exists. The data about the ovulation determination date O_{n(BI)} exists when the user executes the above measuring process of S6 (selects the measuring mode) continuously and an actual ovulation date has already come in the present cycle (a change in the BI value determined in the above S604 has occurred). Therefore, when the data about the ovulation determination date O_{n(BI)} exists, the CPU 35 proceeds to S704 and sets the ovulation date Oₙ of the present cycle as the ovulation determination date O_{n(BI)}· Then, the α^{th} oncoming ovulation date O_{n+α} is estimated by multiplying the average menstrual cycle Tₙ in days by α and adding the product to the ovulation date Oₙ of the present cycle.

Meanwhile, when it has been determined in S703 that the data about the ovulation determination date O_{n(BI)} does not exist, the CPU 35 proceeds from S703 to S705. In this case, in the female physical condition management apparatus 1, the first day of 14 days prior to the start date of menstruation is set as an ovulation date. Thus, the first day of 14 days prior to the start date of the next menstruation can be estimated as the ovulation date. In this case, as the start date of the next menstruation, the next menstruation start date Mₙ₊₁ estimated in S701 may be used. However, in the present embodiment, insofar as the process of estimating the ovulation date Oₙ is concerned, a date obtained by adding the shortest menstrual cycle Tₙ₍ₘᵢₙ₎ updated in the above input processes (S410 to S412) of S4 to the start date Mₙ of menstruation of the present cycle is taken as the next menstruation start date. Further, a date obtained by subtracting 14 days from the date is estimated as the ovulation date Oₙ in the present cycle. In addition, the α^{th} oncoming ovulation date O_{n+α} is estimated by multiplying the average menstrual cycle Tₙ in days by α and adding the product to the ovulation date Oₙ of the present cycle.

The reason why the ovulation date Oₙ of the present cycle is estimated by use of the shortest menstrual cycle Tₙ₍ₘᵢₙ₎ as described above is as follows. That is, since the next menstruation start date Mₙ₊₁ estimated in S701 is obtained by adding the average menstrual cycle Tₙ to the start date Mₙ of menstruation of the present cycle. Thus, an ovulation date estimated based on this may be a date after the ovulation date Oₙ estimated by use of the shortest menstrual cycle Tₙ₍ₘᵢₙ₎ as described above. Accordingly, when the ovulation date is estimated based on the average menstrual cycle Tₙ, an actual ovulation date is more apt to come earlier than the estimated ovulation date as compared with when the ovulation date is estimated based on shortest menstrual cycle Tₙ₍ₘᵢₙ₎. Meanwhile, the user often plans active activity such as traveling during a period until an ovulation date during which her physical condition is relatively good. In this case, if an actual ovulation date comes earlier than an estimated ovulation date, the user reconsiders the plan by, for example, canceling the planned activity. However, since it is an active plan such as traveling, its reconsideration is impossible, and the body is often exposed to an excessive burden. Further, even if the reconsideration is possible, a great degree of discontentment is liable to be incurred. Thus, in the present embodiment, the ovulation date Oₙ is estimated based on the shortest menstrual cycle Tₙ₍ₘᵢₙ₎ so as to suppress the tendency of earlier advent of an actual ovulation date than the estimated ovulation date Oₙ and reduce the possibility of reconsidering a plan of active activity by the user.

Then, in S706, the start date of the preovulatory and postovulatory period POₙ is estimated. In the female physical condition management apparatus 1, the preovulatory and postovulatory period is defined as a period of five days consisting of an ovulation date Oₙ, two days right before the ovulation date Oₙ and two days right after the ovulation date Oₙ. Therefore, the first day of the two days right before the ovulation date Oₙ can be estimated as the start date of the preovulatory and postovulatory period. That is, in S706, a date obtained by subtracting 2 days from the ovulation date Oₙ set in S704 or estimated in S705 is estimated as the start date POₙ of the preovulatory and postovulatory period in the present cycle. Further, the start date PO_{n+α} of the α^{th} oncoming preovulatory and postovulatory period is estimated by multiplying the average menstrual cycle Tₙ in days by α and adding the product to the start date POₙ of the preovulatory and postovulatory period of the present cycle.

Then, in S707, the start date of the PMS preventive period PPₙ is estimated. In the female physical condition management apparatus 1, the PMS preventive period is defined as a period between the end of a preovulatory and postovulatory period and the start of a PMS period. Therefore, the third day after an ovulation date Oₙ can be estimated as the start date of the PMS preventive period. That is, in S707, a date obtained by adding 3 days to the ovulation date Oₙ set in S704 or estimated in S705 is estimated as the start date PPₙ of the PMS preventive period in the present cycle. Further, the start date PP_{n+α} of the α^{th} oncoming PMS preventive period is estimated by multiplying the average menstrual cycle Tₙ in days by α and adding the product to the start date PPₙ of the PMS preventive period of the present cycle.

Then, in S708, the start date of the PMS period Pₙ is estimated. In the female physical condition management apparatus 1, the PMS period is defined as a period of seven days right before menstruation. Therefore, the first day of seven days right before the next menstruation start date Mₙ₊₁ can be estimated as the start date of the PMS period Pₙ. That is, in S708, a date obtained by subtracting 7 days from the next menstruation start date Mₙ₊₁ estimated in S701 is estimated as the start date Pₙ of the PMS period in the present cycle. In addition, the start date P_{n+α} of the α^{th} oncoming PMS period is estimated by multiplying the average menstrual cycle Tₙ in days by α and adding the product to the start date Pₙ of the PMS period of the present cycle.

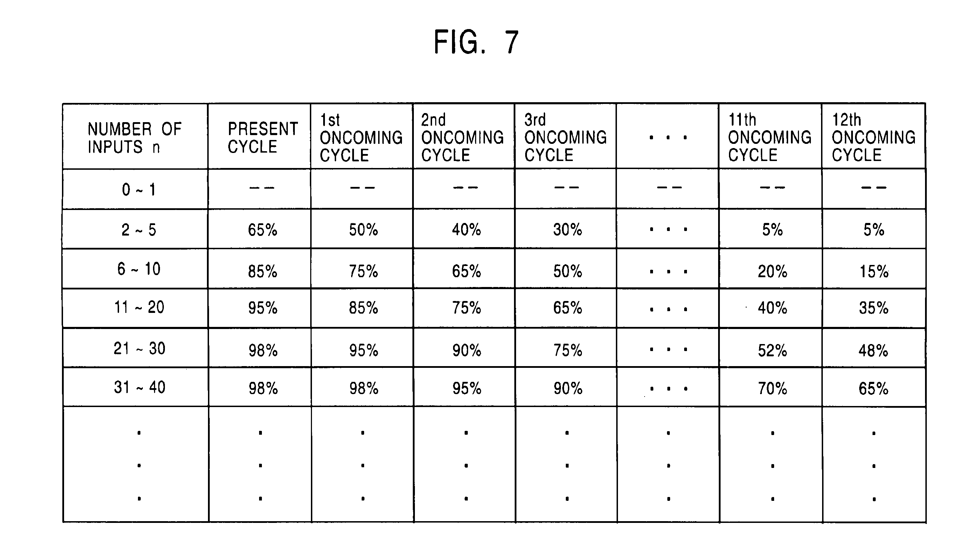

Then, in S709, the accuracy of the estimations made in S701 to S708 are determined. Particularly, in the female physical condition management apparatus 1, a probability that the physical conditions actually appear on the start dates estimated in S701 to S708 is determined. As shown in Fig. 7, as probability data corresponding to the menstruation input number n, probability data with respect to the present cycle and the 1^{st} (next) to 12^{th} oncoming cycles are stored in the memory 34 in advance. Therefore, the CPU 35 can determine the probabilities with respect to the present cycle and the 1^{st} to 12^{th} oncoming cycles by referring to the menstruation input number n counted in the above input process (S403 or S408) of S4. As a matter of course, these probabilities may not only be stored in the memory 34 in advance as probability data as in the present embodiment but also be calculated each time the estimation process is executed by use of a probability calculation formula taking, as variables, the menstruation input number n and "α" which indicates that the estimation is made with respect to the α^{th} oncoming cycle.

By the above S700 to S709, the start dates of the menstrual period, diet period, preovulatory and postovulatory period, PMS preventive period and PMS period in the present cycle and the subsequent cycles are estimated from data about the menstruation start date Mₙ, average menstrual cycle Tₙ and ovulation determination date O_{n(BI)} obtained in S4 or 56 of the main processing. In addition, the probability that the physical conditions actually appear on the estimated start dates is determined based on the number of times the menstruation start date has been input. Further, as a physical condition appearing in a monthly cycle, the time of appearance of a fertile period may also be determined.

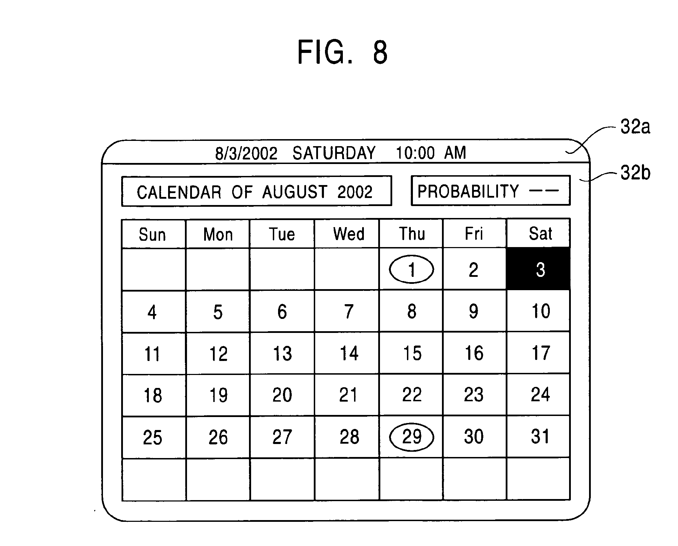

Next, the display process in S8 of the main processing will be described by use of Figs. 8 to 12. In this display process, data about the time of appearance and estimation accuracy read from the memory 34 in S2 or obtained in the estimation process in S7 are displayed on the display screen 32 according to the operation of the operation section 31 by the user.

Firstly, as shown in Fig. 8, the display screen 32 comprises an upper display section 32a which is sectioned in a narrow width along the upper side of the screen viewed from the front side and a lower display section 32b which is the remaining screen portion excluding the upper display section 32a. While the power of the female physical condition management apparatus 1 is on, data about the current date and time are kept displayed in the upper display section 32a, and data selected by the user's operation of the direction button 31d of the operation section 31 is displayed in the lower display section 32b.

Referring to Fig. 8, the screen shown in Fig. 8 is an initial screen to be displayed first when the main processing reaches S8. On this initial screen, in the lower display section 32b, the calendar of one month including the current date is displayed across nearly its whole display area, and a title is also displayed on the upper left side of the month calendar. Further, on the month calendar, the current date is highlighted, and menstruation start dates are displayed in circles. To be more specific, on the initial screen in Fig. 8, "8/3/2002, Saturday, 10:00 AM" is displayed in the upper display section 32a, and in the lower display section 32b, day "3" is highlighted, and a calendar having day "1" and day "29" circled and a title "Calender of August 2002" are displayed.

As for the above menstruation start date, a date read in S2 of the main processing as stored data is displayed immediately after the female physical condition management apparatus 1 is turned on, and a dates updated by S4 and S7 of the main processing are displayed after these processes are executed. However, when no data about the menstruation start dates are stored in the memory 34 and S4 and S7 of the main processing are not yet executed, e.g., when the female physical condition management apparatus 1 is used for the first time, the menstruation start dates are not displayed.

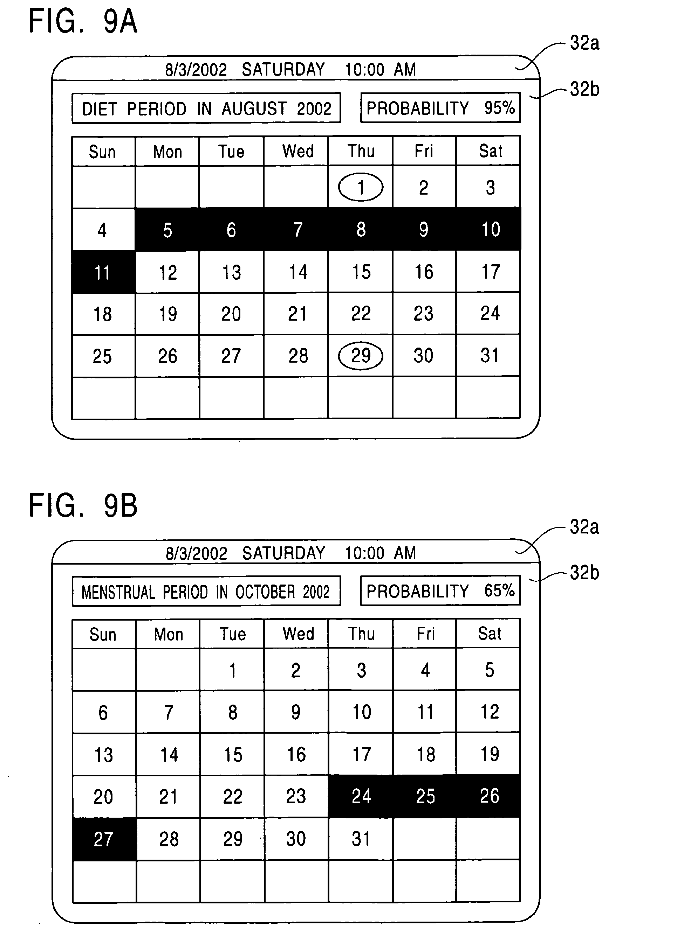

Fig. 9 is a screen displaying data about the times of appearance of physical conditions appearing in a monthly cycle which are estimated by the female physical condition management apparatus 1 and the accuracies of the estimations. On the screen, the calendar of one month starting from day 1 is displayed in a lower display section 32b, and on the month calendar, a period during which a specific physical condition appears is highlighted. As the period of appearance, from the start date of a specific physical condition period to a date right before the start date of the next physical condition period to appear are highlighted based on the start dates of various physical condition periods estimated in the above S7. Further, on the upper left side of the month calendar, the name of the physical condition appearing during the highlighted period is displayed as a corresponding title, and on the upper right side of the month calendar, a probability that the physical condition of the title actually appears during the highlighted period is displayed.

To be more specific, on the screen of Fig. 9A, a period starting from day 5 and ended at day 11 is highlighted on the calendar of August, 2002 including the current date. On the upper left side of the month calendar, "Diet Period in August 2002" is displayed, and on the upper right side of the month calendar, "probability: 95%" is displayed. Further, on the screen of Fig. 9B, a period starting from day 24 and ended at day 27 is highlighted on the calendar of October which is two months ahead of the current date. On the upper left side of the month calendar, "Menstrual Period in October 2002" is displayed, and on the upper right side of the month calendar, "probability: 65%" is displayed.

As for the above period of appearance of the physical condition and probability, the period of appearance and probability read in S2 of the main processing as stored data are displayed immediately after the female physical condition management apparatus 1 is turned on, and the period of appearance and probability updated by S4 and S7 of the main processing are displayed after these processes are executed. However, when these period of appearance and probability are not yet stored in the memory 34 and S4 and S7 of the main processing are not yet executed, e.g., when the female physical condition management apparatus 1 is used for the first time, a message such as "Data is insufficient. Select and execute an input mode." is displayed in place of these period of appearance and probability.

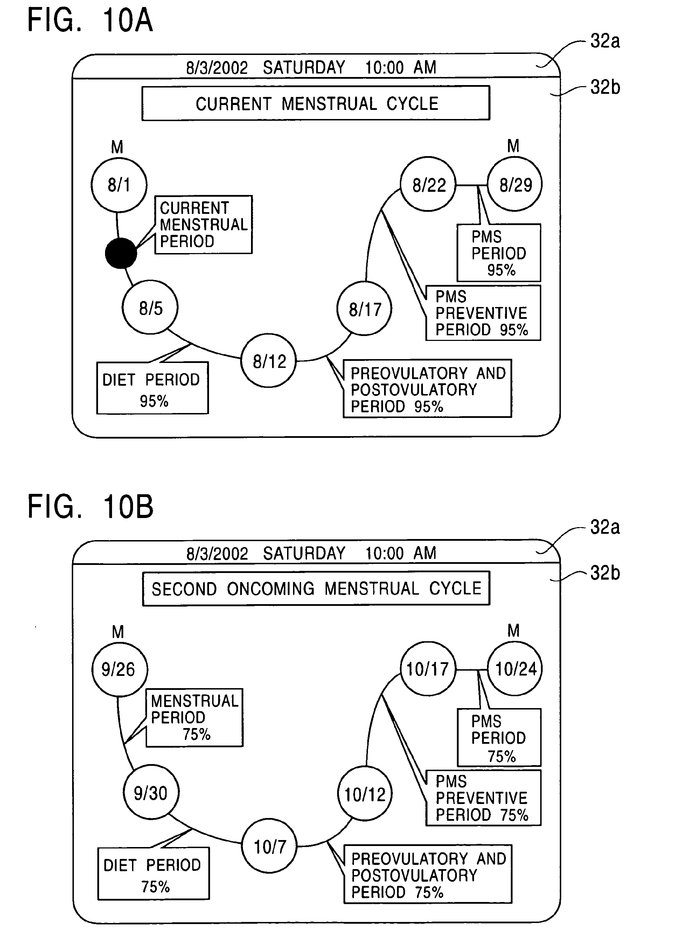

Fig. 10 is another screen displaying data about the times of appearance of a physical condition appearing in a monthly cycle which are estimated by the female physical condition management apparatus 1 and the accuracies of the estimations. In a lower display section 32b on this screen, the calendar of a menstrual cycle starting from the start date of menstruation and ended at the start date of the next menstruation (hereinafter referred to as "cycle calendar" in the present embodiment) is displayed in place of such a month calendar as shown in Fig. 9. Further, on this cycle calendar, the start dates of various physical condition periods appearing within this menstrual cycle are displayed, and the names of the physical condition periods starting on the start dates are displayed together with probabilities that the physical conditions actually appear on the start dates. In addition, on the upper left side of this cycle calendar, a corresponding title indicating that the menstrual cycle on the calendar is the α^{th} oncoming menstrual cycle from the current menstrual cycle is displayed.

To be more specific, in the lower display section 31b on the screen of Fig. 10A, a symbol "8/1" signifying August 1 and a symbol "8/29" signifying August 29 are displayed together with a symbol "M" signifying the start date of menstruation and a curve connecting these date symbols to each other. On this curve, date symbols "8/5", "8/12", "8/17" and "8/22" signifying the start dates of the physical condition periods appearing during this cycle are displayed. Further, together with "95%" which is a probability that the physical condition periods actually appear, the names of the physical condition periods such as "menstrual period", "diet period", "preovulatory and postovulatory period", "PMS preventive period" and "PMS period" are displayed, pointing curve sections sandwiched in between the above date symbols. However, on the curve, "•" is displayed on the position corresponding to August 3 which is the current date, and "present" is displayed in the "menstrual period" to which the current date belongs in place of the probability. Further, "Current Menstrual Cycle" is displayed on the upper side of the cycle calendar. Meanwhile, on the screen of Fig. 10B, "9/26" and "10/24" are displayed as date symbols signifying the start dates of menstruation, and "9/30", "10/7", "10/12" and "10/17" are displayed as date symbols signifying the start dates of the physical conditions. Further, together with the names of the physical condition periods, "75%" which is a probability that the physical condition periods actually appear is displayed. In addition, "Second Oncoming Menstrual Cycle" is displayed on the upper side of this cycle calendar.

As for the above start dates of the physical condition periods and probability, the start dates and probability read in S2 of the main processing as stored data are displayed immediately after the female physical condition management apparatus 1 is turned on, and the start dates and probability updated by S4 and S7 of the main processing are displayed after these processes are executed. However, when these start dates and probability are not yet stored in the memory 34 and S4 and S7 of the main processing are not yet executed, e.g., when the female physical condition management apparatus 1 is used for the first time, a message such as "Data is insufficient. Select and execute an input mode." is displayed in place of these start dates and probability.

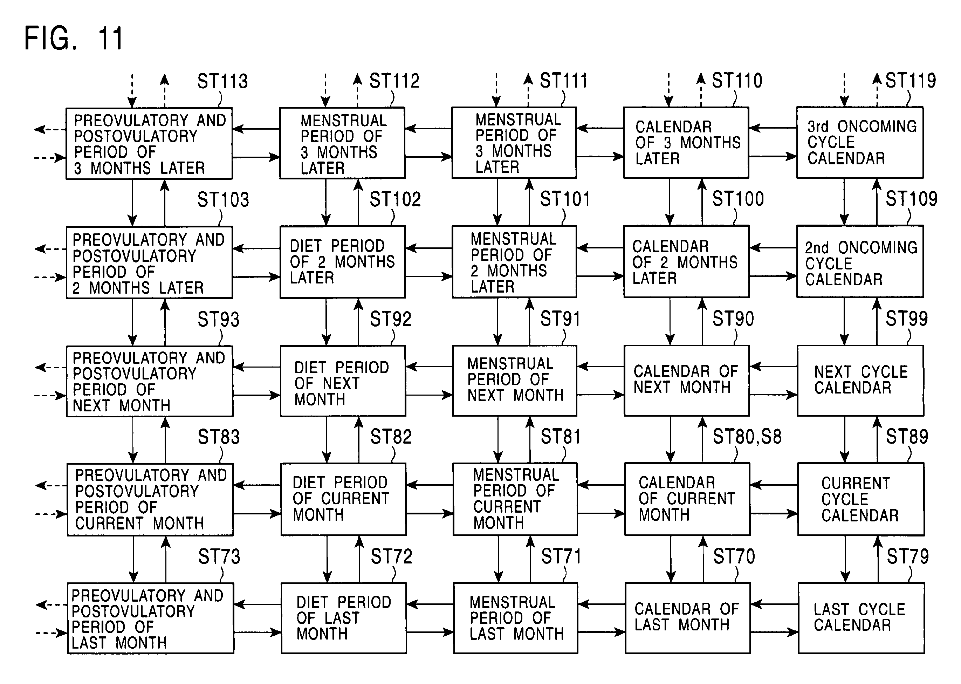

The initial screen of Fig. 8, the screen of Fig. 9 and the screen of Fig. 10 are switched from one to another as appropriate when the user operates the above direction button 31d. The operation of switching the screens will be described by use of the screen display transition diagram shown in Fig. 11.

Firstly, the initial screen of Fig. 8 corresponds to ST80 of Fig. 11. When the UP direction (↑) or DOWN direction (↓) of the direction button is pressed down in ST80, the month calendar and title thereof which are displayed in the lower display section 32b are switched to another month calendar and title. That is, each time the UP direction (↑) of the direction button is pressed down, the month calendar and title thereof are switched to the month calendar of the next month (ST90), the month calendar of two months later (ST100), the month calendar of three months later (ST110) and so on sequentially. When the DOWN direction (↓) of the direction button is pressed down, the month calendar of the previous month (ST70) is displayed.

Further, when the LEFT direction (←) of the direction button 31d is pressed down in ST80, the initial screen is switched to the screen of Fig. 9. That is, each time the LEFT direction (←) of the direction button is pressed down, the period of appearance of each physical condition period and a title and probability corresponding to the physical condition period are displayed in the lower display section 32b in the order of a menstrual period (ST81), a diet period (ST82) and a preovulatory and postovulatory period (ST83) and a PMS preventive period and a PMS period which are not shown. Meanwhile, each time the UP direction (↑) of the direction button 31d is pressed down after the LEFT direction (←) is pressed down, the period of appearance of the menstrual cycle, diet period or other period in each of the subsequent months including the next month and a title and probability corresponding to the period of appearance are displayed in turn (for example, from ST91 to ST93, from ST101 to ST103, from ST111 to ST113). Hence, when the initial screen (ST80) is, for example, "Calender of August 2002" shown in Fig. 8, the screen "Diet Period in August 2002" of Fig. 9A is displayed by pressing down the LEFT direction (←) of the direction button 31d twice (ST82) , and the screen "Menstrual Period in August 2002" of Fig. 9B is displayed by pressing down the LEFT direction (←) once from the initial screen and then pressing down the UP direction (↑) twice (ST101).

Further, when the RIGHT direction (→) of the direction button 31d is pressed down in ST80, the initial screen is switched to the screen of Fig. 10, and in the lower display section 32b, a title corresponding to the cycle calendar is displayed (ST89). Then, each time the UP direction (↑) is pressed down, each of titles corresponding to the next to 12^{th} oncoming cycle calendars is displayed in turn (ST99 to ST119) . Accordingly, when the initial screen (ST80) is, for example, "Calender of August 2002" shown in Fig. 8, "Current Menstrual Cycle" of Fig. 10A is displayed by pressing down the RIGHT direction (→) of the direction button 31d (ST89), and then by pressing down the UP direction (↑) twice, "Second Oncoming Menstrual Cycle" of Fig. 10B is displayed (ST109).

As described above, in the display process of S8 of the main processing, the initial screen of Fig. 8 is displayed first, and each time the user presses down the direction button 31d, the screens shown in Figs. 9 and 10 are switched from one to another and displayed in turn.

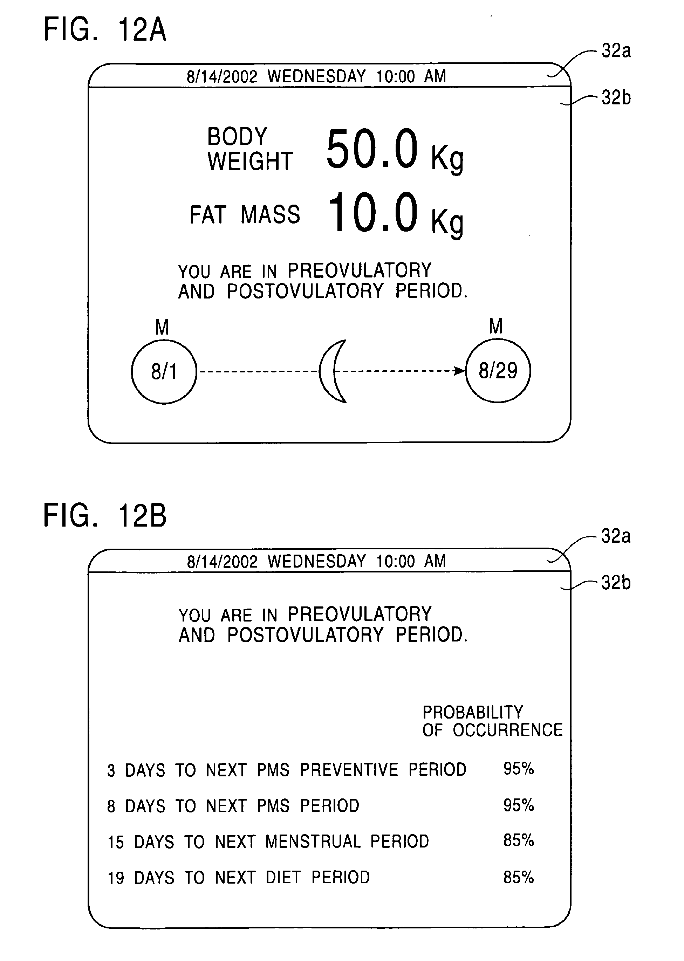

Further, in the female physical condition management apparatus 1 of the present embodiment, only when the CPU has proceeded to the display process in S8 after the measuring mode has been selected in S5 of the main processing and the measuring process in S6 and the estimation process in S7 have been executed, the body weight and body fat mass of the user which have been measured and calculated in S6 are displayed on the display screen 32 as shown in Fig. 12. That is, firstly, as shown in Fig. 12A, data about the current date and time are displayed in the upper display section 32a, and in the lower display section 32b, the body weight and body fat mass of the user which have been measured and calculated in S6 (S601 and S602) are displayed together with data indicating which physical condition period the current date and time belong to. Then, after the screen of Fig. 12A is displayed for a given time (for example, about 15 seconds) , data about which physical condition period the user is currently in, remaining days to the start dates of physical conditions which will appear, and probabilities that the physical conditions actually appear when the days actually pass are displayed in the lower display section 32b as shown in Fig. 12B. Then, after the screen of Fig. 12B is displayed for a given time (for example, about 15 seconds) , the screen of Fig. 12A is displayed again for a given time, and then the screen of Fig. 12B is displayed again. Thus, after the screen of Fig. 12A and the screen of Fig. 12B are displayed for a given time alternately for four times each, the initial screen of Fig. 8 is displayed.

To be more specific, on the screen of Fig. 12A, "8/14/2002 Wednesday 10:00 AM" is displayed in the upper display section 32a, and "body weight: 50.0 kg" and "fat mass: 10.0 kg" are displayed in the lower display section 32b. Further, as the data about which physical condition period the user is currently in, a comment "Your are in the preovulatory and postovulatory period." and a cycle calendar on which the current date is indicated by a crescent mark are displayed. Meanwhile, on the screen of Fig. 12B, a comment "Your are in the preovulatory and postovulatory period." and comments "3 days to the next PMS preventive period. probability of occurrence: 95%", "8 days to the next PMS period. probability of occurrence: 95%", "15 days to the next menstrual period. probability of occurrence: 85%" and "19 days to the next diet period. probability of occurrence:85%" are displayed in the lower display section 32b. As for the probabilities displayed on the screen of Fig. 12B, the probabilities corresponding to the PMS preventive period and the PMS period are different from the probabilities corresponding to the menstrual cycle and the diet period because, in consideration of the current date on the screen, the PMS preventive period and the PMS period are physical conditions appearing in the present cycle and the menstrual cycle and the diet period are physical conditions appearing in the next cycle.

As described above, in the female physical condition management apparatus 1 which is a suitable embodiment of the present invention, the start date of menstruation of a user is input by means of, for example, the operation section 31 which is physical condition data acquiring means, whereby a menstrual cycle is determined, and the BI value of the user is measured by means of, for example, the constant current applying electrodes 21a and 21b, the high frequency constant current circuit 23, the voltage measuring electrodes 22a and 22b and the voltage measuring circuit 24 which are physical condition data acquiring means, whereby an ovulation date is determined. Then, based on the obtained data about physical conditions appearing in a monthly cycle such as the start date of menstruation, menstrual cycle and ovulation date, the start dates of various physical condition periods appearing in a monthly cycle such as a menstrual period, diet period, preovulatory and postovulatory period, PMS preventive period and PMS period are estimated by the CPU 35 which is appearance period estimation means. Further, probabilities that the physical conditions actually appear on the start dates of the physical condition periods which have been estimated by the CPU 35 which is estimation accuracy determination means are determined based on the number of times the above start date of menstruation has been input. Then, the estimated periods of appearance of the physical conditions and start dates of the physical conditions and the determined probabilities are displayed on the display screen 32 which is display means.

A mode for carrying out the present invention is not necessarily limited to the present embodiment. As have been described as appropriate along with the description of the present embodiment, various modifications can be made. Further, the female physical condition management apparatus of the present invention may be used in an embodiment that a user thoroughly inputs data about physical conditions appearing in a monthly cycle or an embodiment that all physical data including a BI value are obtained through measurements. Further, the physical data may be any data correlated with physical conditions of a female which appear in a monthly cycle, such as a basal body temperature. Further, to obtain the BI value, it may be measured not only between both feet of a user but also between both hands, between a hand and a foot or between other specific body parts. Further, other physical data of the user such as a blood pressure and pulse may be further measured and used for obtaining or correcting the above data about the physical conditions. Further, data about the estimation accuracy may be not only displayed as a probability in numeric value but also displayed schematically as a bar graph or by use of a specific number of marks. Alternatively, an abstract comment such as "This estimation is quite reliable." or "This estimation may be wrong." may be displayed. Furthermore, the female physical condition management apparatus of the present invention may not only take the form of an independent unit as in the present embodiment but also be implemented in the form of a network apparatus in which client computers each having a software program for inputting data about physical conditions appearing in a monthly cycle is communicatably connected to a server computer having a software program for estimating the periods of appearance of the physical conditions appearing in a monthly cycle based on the data about the physical conditions and determining the accuracy of the estimation.

As described above, the female physical condition management apparatus of the present invention comprises physical condition data acquiring means, appearance time estimation means, estimation accuracy determination means, and display means, wherein the physical condition data acquiring means acquires data about physical conditions of a female which appear in a monthly cycle, the appearance time estimation means estimates the oncoming times of appearance of the physical conditions appearing in a monthly cycle of the female based on the acquired data about the physical conditions, the estimation accuracy determination means determines the accuracy of the estimations made by the appearance period estimation means, and the display means displays data about the estimated times of appearance and data about the determined estimation accuracy. Thus, the female physical condition management apparatus allows the female user to know how reliable the estimation is and facilitates the user planning activities in the future.

Further, when the estimation accuracy determination means determines the accuracy of the estimations made by the appearance time estimation means based on the number of times the data about the physical conditions appearing in a monthly cycle of the female has been acquired by the physical condition data acquiring means in the past, the estimation accuracy improves as the number of times the data about the physical conditions has been acquired increases. This can cause the female user to feel that repeated use of the female physical condition management apparatus is advantageous and enjoyable and to have the feeling of using the apparatus continuously.

Further, when the data about the estimation accuracy which is displayed by the display means is a probability that the physical conditions appearing in a monthly cycle of the female actually appear at the estimated times of appearance, the female user can easily and clearly know how reliable the estimation is and plan activities in the future more easily.

Further, the physical condition data acquiring means may be used by the female to input the data about the physical conditions appearing in a monthly cycle. In this case, the female user uses the data about the physical conditions which has been acquired by use of, for example, a known female basal body thermometer in this female physical condition management apparatus so as to know the oncoming times of appearance of the physical conditions appearing in a monthly cycle and estimation accuracy thereof.

Alternatively, the physical condition data acquiring means may measure physical data of the female and acquire the data about the physical conditions appearing in a monthly cycle of the female based on the measured physical data. In this case, the female user can acquire the data about the physical conditions by the female physical condition management apparatus and know the oncoming times of appearance of the physical conditions appearing in a monthly cycle and estimation accuracy thereof.

Particularly, the physical data of the female may be the basal body temperature or bioelectric impedance of the female. The data about the physical conditions appearing in a monthly cycle of the female can be acquired regardless of whether the physical data is the basal body temperature or the bioelectric impedance. The oncoming times of appearance of the physical conditions appearing in a monthly cycle of the female can be estimated and the accuracy of the estimation can be determined based on the physical condition data.

Further, the data about the physical conditions appearing in a monthly cycle of the female which is acquired by the physical condition data acquiring means may be the start date of menstruation, ovulation date or menstrual cycle of the female. The oncoming times of appearance of the physical conditions appearing in a monthly cycle of the female can be estimated and the accuracy of the estimation can be determined based on the physical condition data, regardless of whether the physical condition data is the start date of menstruation, the ovulation date or the menstrual cycle.

## Claims

1. A female physical condition management apparatus comprising:
physical condition data acquiring means,
appearance time estimation means,
estimation accuracy determination means, and
display means,
wherein
the physical condition data acquiring means acquires data about physical conditions of a female which appear in a monthly cycle, the appearance time estimation means estimates the oncoming times of appearance of the physical conditions appearing in a monthly cycle of the female based on the acquired data about the physical conditions,
the estimation accuracy determination means determines the accuracy of the estimations made by the appearance period estimation means, and
the display means displays data about the estimated times of appearance and data about the determined estimation accuracy.

2. The apparatus of claim 1, wherein the estimation accuracy determination means determines the accuracy of the estimations made by the appearance time estimation means based on the number of times the data about the physical conditions appearing in a monthly cycle of the female has been acquired by the physical condition data acquiring means in the past.

3. The apparatus of claim 1 or 2, wherein the data about the estimation accuracy which is displayed by the display means is a probability that the physical conditions appearing in a monthly cycle of the female actually appear at the estimated times of appearance.

4. The apparatus of claims 1 to 3, wherein the physical condition data acquiring means is used by the female to input the data about the physical conditions appearing in a monthly cycle.

5. The apparatus of claims 1 to 3, wherein the physical condition data acquiring means measures physical data of the female and acquires the data about the physical conditions appearing in a monthly cycle of the female based on the measured physical data.

6. The apparatus of claim 5, wherein the physical data of the female is the basal body temperature of the female.

7. The apparatus of claim 5, wherein the physical data of the female is the bioelectric impedance of the female.

8. The apparatus of claims 1 to 7, wherein the data about the physical conditions appearing in a monthly cycle of the female which is acquired by the physical condition data acquiring means is the start date of menstruation of the female.

9. The apparatus of claims 1 to 7, wherein the data about the physical conditions appearing in a monthly cycle of the female which is acquired by the physical condition data acquiring means is the ovulation date of the female.

10. The apparatus of claims 1 to 7, wherein the data about the physical conditions appearing in a monthly cycle of the female which is acquired by the physical condition data acquiring means is the number of days in the monthly cycle of the female.
